(19) Europäisches Patentamt
European Patent Office
Office européen des brevets

(11) Publication number: **0 369 035 A1**

(12) # EUROPEAN PATENT APPLICATION published in accordance with Art. 158(3) EPC

(21) Application number: 89906452.1

(22) Date of filing: 06.06.89

(86) International application number: PCT/JP89/00567

(87) International publication number: WO 89/11852 (14.12.89 89/29)

(51) Int. Cl.[5]: A61K 31/165, A61K 31/195, A61K 31/235, A61K 31/44, A61K 31/505

(30) Priority: 06.06.88 JP 139135/88

(43) Date of publication of application: 23.05.90 Bulletin 90/21

(84) Designated Contracting States: BE CH DE FR GB IT LI NL SE

(71) Applicant: SHOWA DENKO KABUSHIKI KAISHA
13-9, Shiba Daimon 1-chome
Minato-ku, Tokyo 105(JP)

Applicant: Okamoto, Shosuke
15-18, Asahigaoka 3-chome Tarumi-ku
Kobe-shi Hyogo(JP)

(72) Inventor: KATSUURA, Yasuhiro
Showa Denko K. K. Seikagaku Kenkyusho
2-24-25, Tamagawa Ota-ku Tokyo 146(JP)
Inventor: OHNO, Norio
Showa Denko K.K. Seikagaku Kenkyusho
2-24-25, Tamagawa Ota-ku Tokyo 146(JP)
Inventor: YAMADA, Morihiko
Showa Denko K. K. Seikagaku Kenkyusho
2-24-25, Tamagawa Ota-ku Tokyo 146(JP)
Inventor: KIMURA, Yoshio
Showa Denko K. K. Seikagaku Kenkyusho
2-24-25, Tamagawa Ota-ku Tokyo 146(JP)
Inventor: OKUNOMIYA, Akiko
13-2, Nakayamatedori 7-chome Chuo-ku
Kobe-shi Hyogo 650(JP)
Inventor: WANAKA, Keiko
1-1, Yokoo 8-chome Suma-ku
Kobe-shi Hyogo 654-01(JP)

(74) Representative: Fisher, Bernard et al
Raworth, Moss & Cook 36 Sydenham Road
Croydon Surrey CR0 2EF(GB)

(54) **AGENT FOR TREATING PANCREATITIS OR THE LIKE.**

EP 0 369 035 A1

(57) The invention relates to an agent for treating pancreatitis or the like, which contains as an active ingredient a compound represented by general formula (I) or a pharmaceutically acceptable salt thereof, wherein A represents (a) or (b), one of $X_1$ and $X_2$ represents a hydrogen atom and the other represents a hydrogen atom, a nitro group, a phenoxy group (optionally substituted by nitro or halogen), a benzyloxy group, a picolyloxy group or a pyridyloxy group (optionally substituted by nitro), and Y represents a $C_1$-$C_6$ alkyl group, a phenyl group (optionally substituted by $C_1$-$C_4$ alkylcarbonyl, phenylcarbonyl, carboxyl or $C_1$-$C_4$ alkyl optionally substituted by carboxyl or $C_1$-$C_4$ alkyl), a pyridyl group (optionally substituted by halogen) or a pyrimidyl group.

TITLE MODIFIED
see front page

DESCRIPTION

TITLE OF THE INVENTION

Theraputic Agent For Pancreatitis and the Like

FIELD OF THE ART

The present invention relates to therapeutic agents for pancreatitis, the disseminated intravascular coagulation syndrome (hereinafter abbreviated as DIC), and shock as well as multiple organ failure (hereinafter abbreviated as MOF) accompanying same.

BACKGROUND OF THE ART

Among known diseases due to and worsened by an abnormally activated proteolytic enzymes in the body, are pancreatitis, DIC, and shock and the like.

As a pathogenesis of pancreatitis Klebs (1868) listed pancreatic enzymes, and subsequently, Chiari (1896) introduced a concept of autolysis by pancreatic enzymes. The autolysis of pancreas in the case of pancreatitis relies heavily on proteolytic enzymes in pancreatic juce. Namely, pancreatitis is generated by an activation of a small amount of trypsinogen in the pancreas to trypsin by pancreatitis-generating factors (cholangio-disease, wounds, severe alcoholism, vascular injuries, metabolic disorders, infections and the like) followed by an activation of kallikrein, elastase, phospholipase A2 and the like, resulting in the autolysis of pancreas. Moreover, the activated enzymes systemically circulate and are involved in the occurrence of systemic disorders observed in pancreatitis. Namely, it is considered that pancreatic enzymes generate DIC directly or via an activation of enzymes in the blood coagulation system or fibrinolytic system resulting in shock, respiratory disorders, renal disorders, liver disorders, and bleeding of the digestive canal, which worsen the prognosis of pancreatitis. Therefore, taking into account the participation of the pancreatic enzyme in the symptom in

pancreatitis, an administration of a pancreatic enzyme inhibitor is expected to be effective for the therapy of pancreatitis.

DIC is a general term for a syndrome wherein blood coagulation factors are activated by pancreatitis, shock, malignant tumors, infections, obstetrical disease, hepatitis, burns, and other diseases, a large number of microthrombuses are formed in small vessels, whereby coagulation factors such as platelets, plasma kallikrein, fibrinogen and the like are consumed, and fibrinolytic enzymes are activated, resulting in severe bleeding. Accordingly, inhibitors of blood coagulation enzymes or fibrinolytic enzymes are expected to be effective for the therapy of DIC. Moreover, a problem is noted that DIC is accompanied by a generation of necrotic region in various organs, especially the lungs, kidney, heart, liver, brain, pituitary and the like, resulting in disorders in the functioning of these organs.

Shock is clinically defined as a condition wherein the blood circulation in tissue is reduced, resulting in a systemic intracellular hypoxia and damage to major organs, and it is known that proteolytic enzymes cause a worsening of the symptoms; particularly, shock due to DIC based on an infection, shock due to an operation or wound, shock due to burns, and shock caused by an allergic reaction are known. As a symptomatic treatment for shock, an administration of steroids is mentioned, but since it is known that steroids are disadvantageous in that they cause severe damage to the adrenal cortex functions, and result in a formation of peptic ulcers, an elevation of the blood sugar and the like, as side effects, the development of a safe anti-shock agent is urgently required.

The development of enzyme inhibitors having the above-mentioned action has been carried out for many years, and among such inhibitors used as agents which

exhibit pharmacological actions somewhat similar to those of the present invention, aprotinin (Trasylol®), gabexate mesilate (FOY®) nafamostat mesilate (FUT-175, Futhan®) and the like are mentioned, but these inhibitors have problems of effects and safety. For example, aprotinin is a proteolytic enzyme inhibitor which is a peptide extracted from a bovine lung, pancreas, or parotit glaud, and therefore, becomes autigen when administered to a human, and a continuous administration thereof may cause side effects such as allergy, shock, and the like. Gabexate mesilate inhibits not only trypsin but also thrombin and plasmin and thus is promising as an effective agent for the therapy of not only pancreatitis but also the DIC and the like. This agent, however, is unstable in vivo, and thus a large amount must be administered to obtain the expected therapeutic effect, resulting in a risk of side effects such as inflammation of vessels. Nafamostat mesilate exhibits an enzyme inhibitory activity stronger than that of gabexate mesilate, and is used to alleviate acute symptoms in pancreatitis. Nevertheless, this agent must be carefully monitored during clinical use thereof, due to its enzyme inhibitory spectrum, short in vivo half life, strong hypotensive action, and low 50% lethal dose (hereinafter abbreviated as $LD_{50}$) when administered to an animal, thus increasing the load on patients and doctors. Since the symptoms such as pancreatitis, the DIC, shock and the like progress very rapidly, and frequently cause a complication of the MOF, the possibility of death of the patients is high unless an appropriate therapy is carried out. Accordingly, for an internal treatment of patients, the development of enzyme inhibitors which and promise to provide sharp and reliable effects, is desired.

An object of the present invention is to solve the above-mentioned conventional problems and to provide a medicament effective for more essential therapy, has a

long half life in vivo, and has a high $LD_{50}$ value, resulting in an easier use thereof.

BEST MODE OF CARRYING OUT THE INVENTION

After intensive research into the development of a medicament effective for the therapy of pancreatitis, DIC, shock, and MOF, the present inventors found that phenylalanine derivatives represented by the following formula exhibit excellent therapeutic effects, and thus accomplished the present invention.

The present invention is intended to provide a pharmaceutical preparation for pancreatitis and the like containing, as an effective ingredient, a compound represented by the general formula:

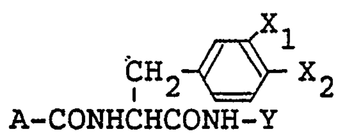

wherein A represents $H_2NCH_2$ 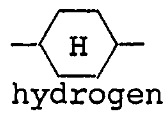 or $H_2NCH_2$ 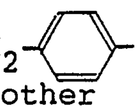 ; one of $X_1$ and $X_2$ represents hydrogen and the other represents hydrogen, nitro, phenoxy (optionally substituted by nitro or by halogen), benzyloxy, picolyloxy or pyridyloxy (optionally substituted by nitro); and Y represents $C_1$-$C_6$ alkyl, phenyl (optionally substituted by $C_1$-$C_4$ alkyl carbonyl, by phenylcarbonyl, by carboxyl, or by $C_1$-$C_4$ alkyl optionally substituted by carboxyl or $C_1$-$C_4$ alkoxycarbonyl), pyridyl (optionally substituted by halogen), or pyrimidyl, or pharmaceutically acceptable salts thereof.

As the above-mentioned pharmaceutically acceptable salts, for example, inorganic salts such as hydrochloride, hydrobromide, hydroiodide, sulfate, nitrate, phosphate and the like, and organic salts such as oxalate, succinate, maleate, glycolate, malate, citrate, lactate, benzenesulfonate, toluenesulfonate, and methanesulfonate are mentioned. Note, the processes for the production of the compounds represented by the above-mentioned general formula are disclosed in Japanese Unexamined Patent Publication (KOKAI) Nos.

63-22031 and 63-239256.

Typical compounds represented by the above-mentioned general formula (I) are exemplified in Table 1, and their properties also described. Note, the compounds exemplified in the Table are numbered, and as a matter of convenience, hereinafter individual compounds will be identified by the corresponding numbers.

Where the present phenylalanine derivative or a pharmaceutically acceptable salt thereof is used as a therapeutic agent for pancreatitis, DIC, shock, MOF or the like, it is injected or infused by mixing in a conventional manner with an injective medium or supplemental fluid, optionally after mixing with an appropriate additive, or is orally administered as a tablet, capsule, granules, fine granules, powders or the like. The applied dosage is determined in accordance with the age and body weight of, and severity of the symptoms shown by a patient. Namely, when injected or infused, the daily dose for an adult is approximately 1 mg to 300 mg, and when orally administered, the daily dose for an adult is 10 mg to 1000 mg.

When the present compound is used as therapeutic agent for pancreatitis, DIC, shock or MOF, for example, it is formulated as follows.

Example 1. Injective agent for infusion

| | | |
|---|---|---|
| (1) | The present compound | 1.0 g |
| (2) | Sodium chloride | 6.0 g |
| (3) | Calcium chloride | 0.4 g |
| (4) | Injective distilled water | q.s. |
| | Total | 1000.0 ml |

The above-mentioned (1) to (3) are thoroughly dissolved in injective distilled water, the solution is distributed to ampoules, and the ampoules are sealed and sterilized at 121°C for 20 minutes. Alternatively, the solution is distributed to ampoules, and after sterilization in a similar manner , they are lyophilized

Example 2. Injective agent for infusion

| | | |
|---|---|---|
| (1) | The present compound | 1.0 g |
| (2) | Glucose | 13.6 g |
| (3) | Sodium chloride | 6.0 g |
| (4) | Calcium chloride | 0.25 g |
| (5) | Injective distilled water | q.s. |
| | Total | 1000.0 ml |

The above-mentioned (1) and (2) are thoroughly dissolved in injective distilled water, and then (3) and (4) are added and dissolved therein. The solution is distributed to ampoules, and the ampoules are sealed and sterilized at 115°C for 30 minutes. Alternatively, the solution is distributed to ampoules and after sterilization in the same manner as above, they are lyophilized.

Example 3. Tablets

| | | |
|---|---|---|
| (1) | The present compound | 100 g |
| (2) | Lactose | 13 g |
| (3) | Carboxymethylcellulose | 4 g |
| (4) | Hydroxypropyl magnesium | 2 g |
| (5) | Magnesium stearate | 1 g |
| | 1000 tablets | 120 g |

All of the components are mixed, and the mixture then pressed by a tabletizing machine to prepare 1000 tablets containing 100 mg/tablet of the ingredient (1).

The fact that the present phenylalanine derivatives or pharmaceutical salts thereof exhibit strong protease inhibitory activity is illustrated thereinafter by typical tests for anti-plasmin activity, anti-kallikrein activity, anti-trypsin activity, anti-urokinase activity, and anti-blood coagulation factor 10 (FXa) activity. Moreover, the effectiveness thereof for the therapy of acute pancreatitis, DIC, shock, and MOF is clearly confirmed in the Examples, using experimental animals, as described hereinafter.

[I] Assay of enzyme inhibitory activity

1) Assay of anti-plasmin activity

i) Assay method of inhibition of

fibrinolysis

A test substance was dissolved in 0.18 M borate-physiological saline buffer (pH7.4) to make a total amount of 600 µl, and to the solution were added 200 µl of 0.2% bovine fibrinogen dissolved in the same buffer, 100 µl of 0.3 casein units/ml human plasmin solution, and 100 µl of 50 units/ml bovine thrombin solution, and the whole incubated in an incubator at 37°C, and then a time necessary for lysis of the formed fibrin clot was measured, and a concentration of the test compound, $I_{50}$ (concentration for 50% inhibition in unit µ mol) which provides a lysis time of the fibrin clot twice as long as that provided by a control run in which the test substance was not used, (in this experiment, about 5 minutes) was obtained.

ii) Assay method of inhibition of S-2251 degradation

The test substance was dissolved in a 0.05 M Tris-HCl (pH7.4) buffer to make a total volume of 400 µl, and 50 µl of a 3 mM S-2251 solution was added to the solution. The mixture was incubated at 37°C for 5 minutes in an incubator, and after the addition of 50 µl of 0.2 casein units/ml of human plasmin, and incubation at 37°C for 4 minutes, the reaction was terminated by an addition of 50 µl of 50% acetic acid. An absorbance at 405 nm of 4-nitroaniline formed in the reaction system was measured to obtain a concentration (µ mole) of the test substance, $I_{50}$ , which showed an absorbance of one half of that of a control in which the test substance was not used.

2) Assay of anti-plasma kallikrein activity

Assay method of inhibition of S-2302 degradation

The test substance was dissolved in a 0.05 M Tris-HCl (pH 7.8) buffer to make a total volume of 400 µl, and 50 µl of a 2 mM S-2302 solution was added to the solution. After incubation at 37°C for 5 minutes in

an incubator, an addition of 50 μl of 0.12 units/ml human plasma kallikrein solution, and incubation at 37°C for 5 minutes, the reaction was terminated by an addition of 50 μl of 50% acetic acid. An absorbance at 405 nm of 4-nitroaniline formed in the reaction system was measured to obtain a concentration (μmole) of the test substance, $I_{50}$ , which showed an absorbance of one half of that of a control in which the test substance was not used.

3) Assay of anti-urokinase activity

Assay method of inhibition of S-2444 degradation

The test substance was dissolved in a 0.05 M Tris-HCl (pH 8.8) buffer to make a total volume of 400 μl, and 50 μl of a 1 mM S-2444 solution was added to the mixture. After incubation at 37°C for 5 minutes in an incubator, an addition of 50 μl of 500 units/ml human urokinase solution, and incubation at 37°C for 5 minutes, the reaction was terminated by an addition of 50 μl of 50% acetic acid. An absorbance at 405 nm of 4-nitroaniline formed in the reaction system was measured to obtain a concentration (μmole) of the test substance, $I_{50}$ , which showed an absorbance of one half of that of a control in which the test substance was not used.

4) Assay of anti-FXa activity

Assay method of inhibition of S-2222 degradation

To 0.4 ml of the test substance dissolved in a 0.05 M Tris-HCl (pH 8.3) buffer is added 0.05 ml of 3 mM S-2222 solution, and after incubation at 37°C for about 4 minutes, an addition of 0.05 ml of 0.3 vial/ml bovine factor Xa, and a reaction at 37°C for 4 minutes, the reaction was terminated by adding 0.05 ml of 50% acetic acid. An absorbance at 405 nm of 4-nitroaniline formed in the reaction system to obtain a concentration of the test substance, $I_{50}$ (μmole), which showed an absorbance

of one half of that of a control in which the test substance was not used.

The experimental results are shown in Table 2. In addition, the names and structures of existing compounds are shown in Table 3-1 as comparative examples, and the experimental results are shown in Table 3-2.

[II] Effect on experimental pancreatitis

SD-rats, body weight of about 200 g were, starved overnight, and 10 to 24 animals were used in one group. Sodium pentobarbital was intraperioneally administered to anesthetize each rat, and then a cannula was inserted to the femoral vein for the administration of the test substance. After abdominal surgery, the bile duct was blocked at a part thereof proximate to the liver, by a clip, a polyethylene tube was inserted to the common bile duct at a part thereof most proximate to the duodenum, and 0.1 ml of solution containing 2 mg sodium taurochorate and 1000 U trypsin dissolved in physiological saline was injected retrogrodely. After removal of the clip and tube, the common bile duct was ligated and the abdomen was closed. Note, the test substance was dissolved in an injective 5% glucose solution, and in the case of a continuous infusion, the solution was administered over 80 minutes starting from 10 minutes before the generation of pancreatitis. In the case of oral administration, the test substance was administered through an oral catheter 30 minutes before the generation of pancreatitis. After 24 hours and 48 hours from the generation of pancreatitis, the survival ratio of the animals was calculated and compared with a control group. The experimental results are shown in Tables 4-1, 4-2, and 4-3.

[III] Effect on experimental DIC

SD-rats, body weight of about 200 g, were starved overnight. Sodium pentobarbital was intraperioneally administered to anesthetize a rat, and

then a cannula was inserted to the femoral vein, and the test substance dissolved in an injective 5% glucose solution was continually infused. Then, 10 minutes from the start of the injection, endotoxin (E. coli 0127:B8, Difco, U.S.) dissolved in physiological saline was injected through the tail vein to an amount of 10 mg/kg body weight, and 4 hours from the administration of endotoxin, the abdomen was opened, blood was obtained from the abdominal aorta, and platelets (Plt), fibrinogen (Fbg), fibrin degradation product (FDP), glutamic oxaloacetic transamirase (GOT), creatinine phosphokinase (CPK), and blood urin nitrogen (BUN) were measured. The test results are shown in Tables 5-1 and 5-2.

[IV] Effect on experimental shock

ICR-mice, body weight of about 25 g, were starved overnight. The test substance was dissolved in an injective 5% glucose solution and intraperioneally administered, and 15 minutes later, endotoxin (E. coli 0127:B8, Difco, U.S.) dissolved in a physiological saline was intraperioneally administered to an amount of 40 mg/kg body weight. After 24 hours from the endotoxin administration, the animals were observed to determine a survival ratio. The experimental results are shown in Tables 6-1 and 6-2.

[V] Acute toxicity test

ICR male mice 5 to 10 weeks old were used. The test substance was dissolved in a 5% dimethylsulfoxide aqueous solution, and the solution was intravenously administered in an amount of 0.1 to 0.2 ml/10 g body weight. An $LD_{50}$ value was calculated by up-and-down method. The experimental results are shown in Tables 7-1 and 7-2.

[VI] Measurement of half life in blood

SD-rats 6 to 7 weeks old were used. The test substance was dissolved in an injective 5% glucose solution and administered to the tail vein. After the

administration, blood samples were periodically obtained from the great vein and placed into heparinized test tubes. Plasma was separated by centrifugation and the test substance was extracted with ethyl acetate. After removal of the solvent, the residue was dissolved in 200 μl of methanol, and analyzed by high performance liquid chromatography. From the change of the concentration of the test substance in blood with time, a half life thereof in blood was calculated. The results are shown in Tables 8-1 and 8-2.

As shown in Table 2, compounds listed in Table 1 exhibited a strong inhibitory action on plasmin, trypsin, plasma kallikrein, FXa, and the like. A strong effect was exhibited on experimental pancreatitis and shock, in comparison with existing control drugs, as shown in Tables 4-1, 4-2, 4-3, 6-1, and 6-2. Moreover, as shown in Tables 5-1 and 5-2, in experimental DIC, the present compounds not only strongly inhibit the activation of the blood coagulation system and fibrolytic system, at a dose lower than existing control drugs, but also alleviate complicated organ disorders. In addition, as shown in Table 7-1, the present compounds exhibit an $LD_{50}$ value of at least 50 to 100 mg/kg, and have a broader safety margin than that of existing control drugs. Additionally, Tables 8-1 and 8-2 show that compounds selected from the compounds listed in Table 1 have a half life in blood longer than that of existing control drugs. Therefore, as seen from the above, the present compounds, in comparison with existing control drugs, are extremely safe and easy to use as therapeutic agent for pancreatitis, DIC, shock, and MOF.

Table 1-1

| No. | Compound | |
|---|---|---|
| 1 | 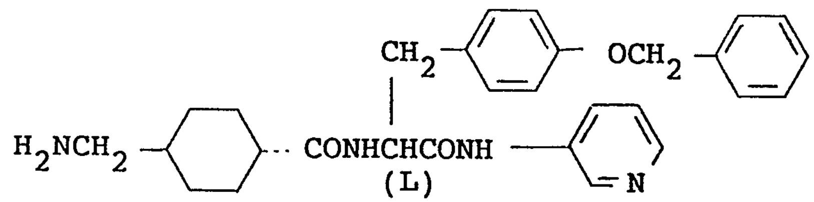 | 2HCl |
| 2 | 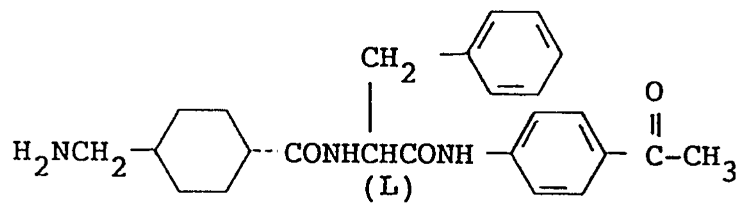 | HCl |
| 3 | 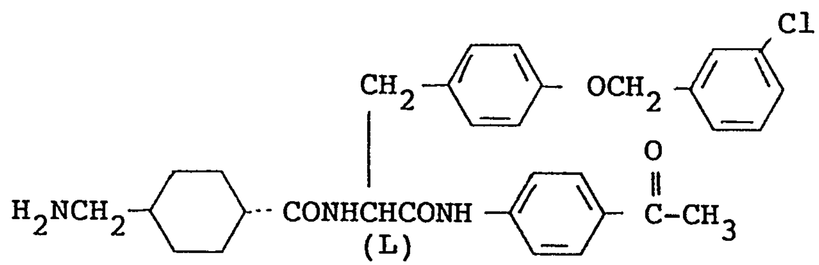 | $CH_3SO_3H$ |
| 4 | 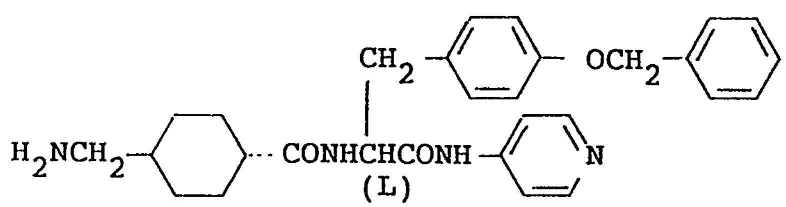 | 2HCl |

Table 1-1 (continued)

| No. | Compound | |
|---|---|---|
| 5 | 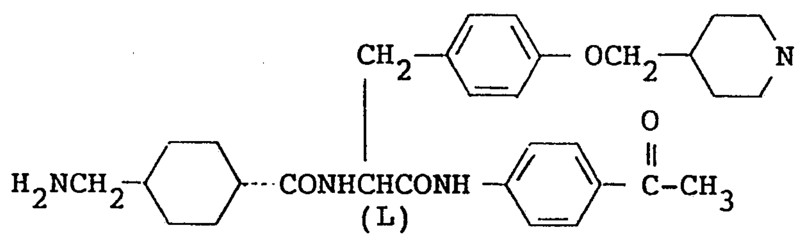 | 2HCl |
| 6 | 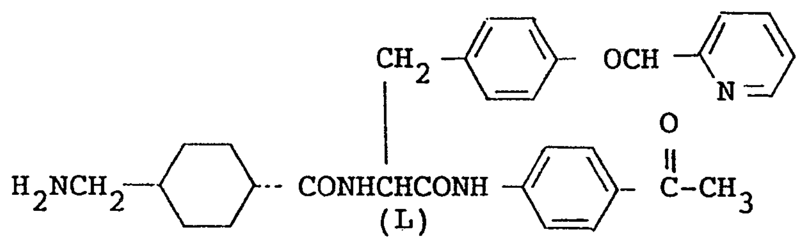 | 2HCl |
| 7 | 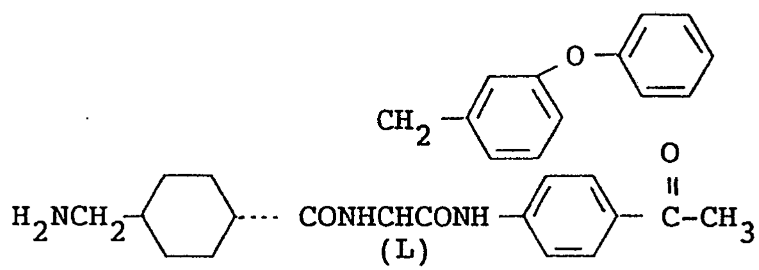 | HCl |
| 8 | 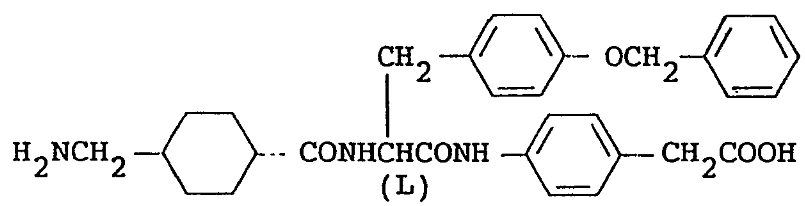 | HCl |

Table 1-1 (continued)

| No. | Compound | |
|---|---|---|
| 9 | 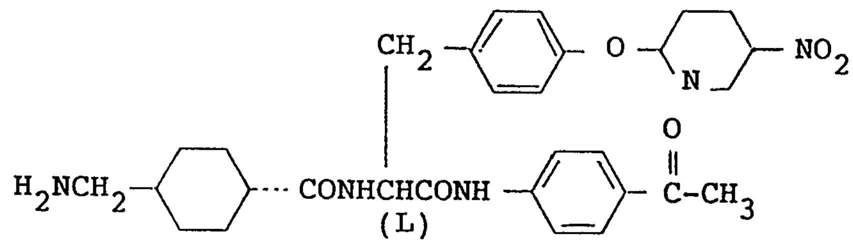 | 2HCl |
| 10 | 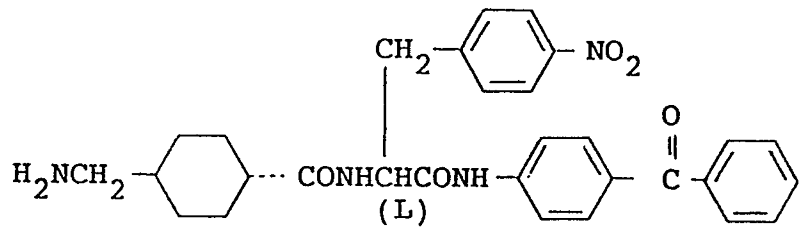 | $CH_3SO_3H$ |
| 11 | 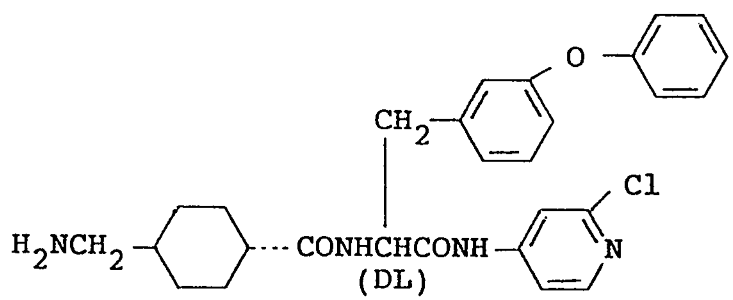 | 2HCl |

Table 1-1 (continued)

| No. | Compound | |
|---|---|---|
| 12 | 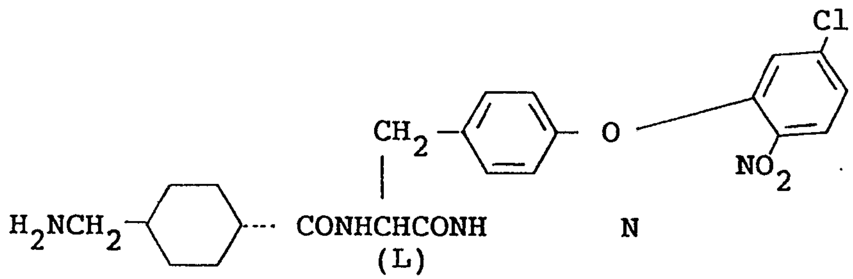 | 2HCl |
| 13 | 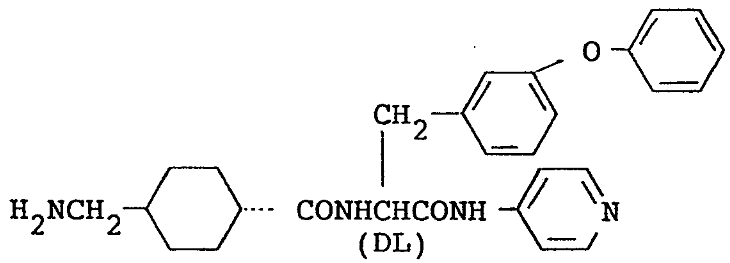 | 2HCl |
| 14 | 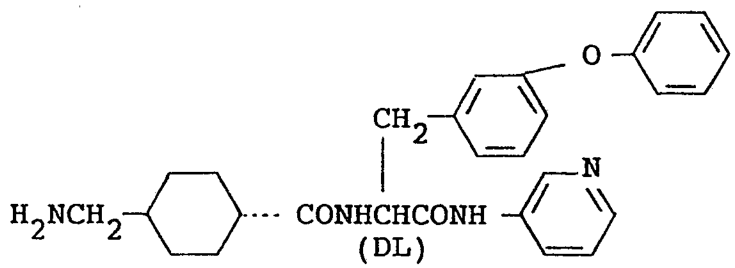 | 2HCl |

Table 1-1 (continued)

| No. | Compound | |
|---|---|---|
| 15 | 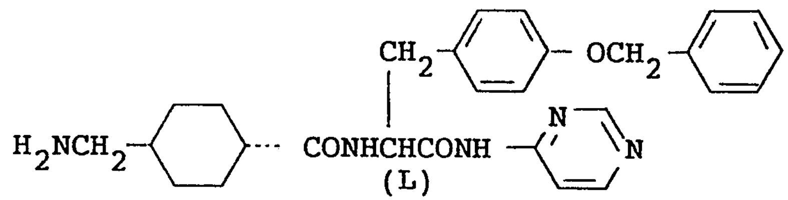 | 2HCl |
| 16 | 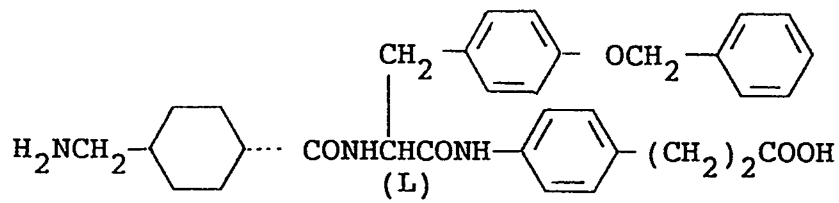 | HCl |
| 17 | 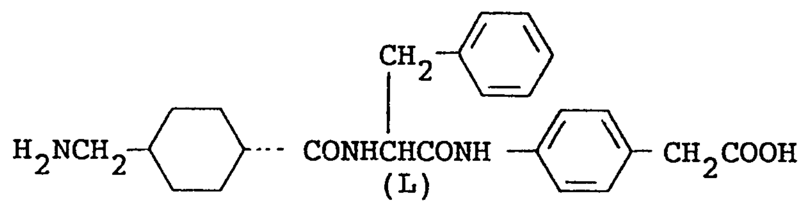 | HCl |
| 18 | 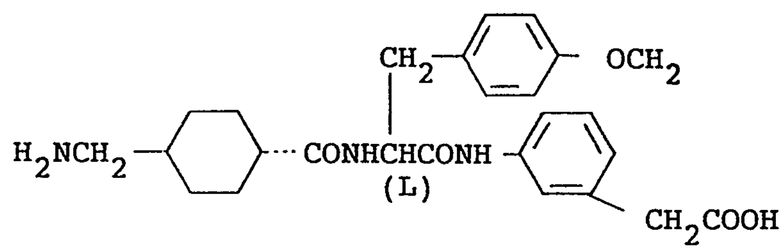 | HCl |

Table 1-1 (continued)

| No. | Compound | |
|---|---|---|
| 19 | 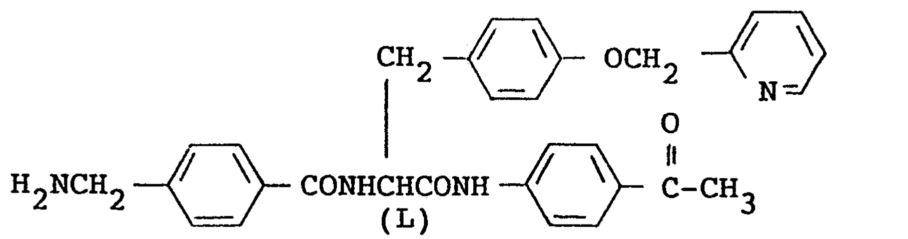 | HCL |
| 20 | 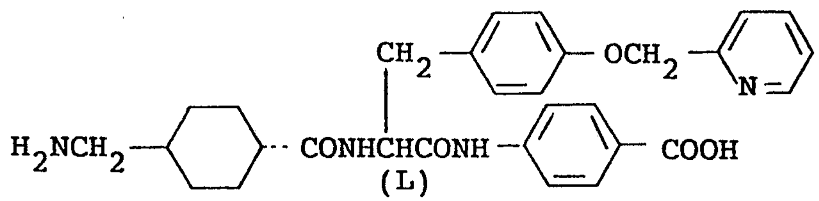 | 2HCl |
| 21 | 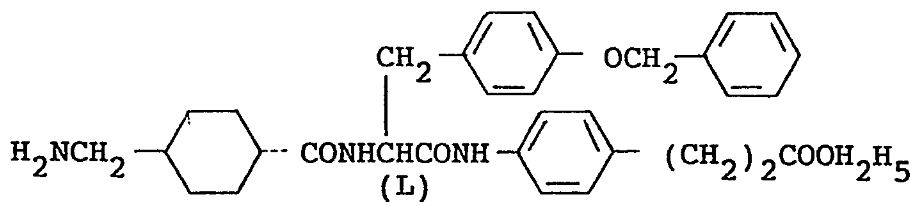 | HCl |
| 22 | 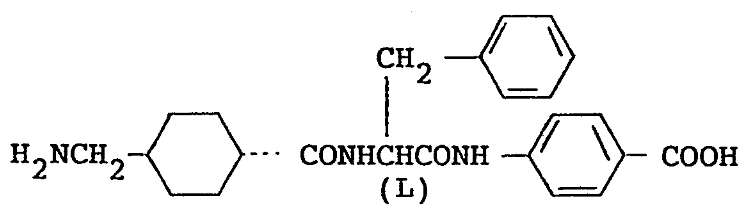 | HCl |

Table 1-1 (continued)

| No. | Compound | |
|---|---|---|
| 23 | 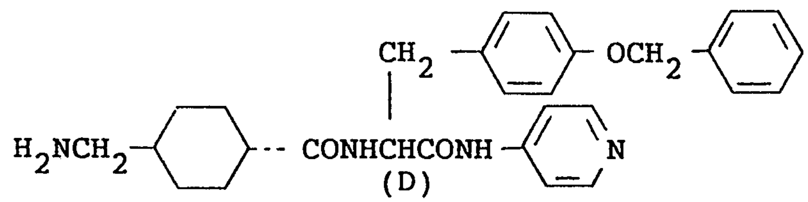 | 2HCl |
| 24 | 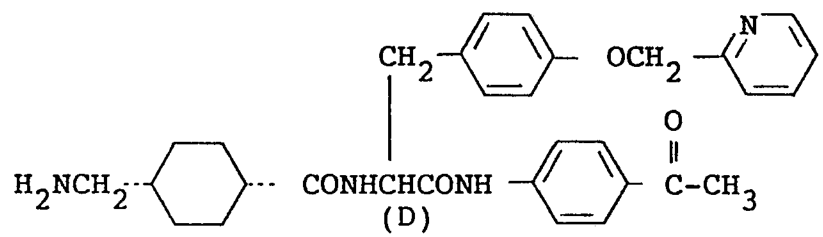 | 2HCL |
| 25 | 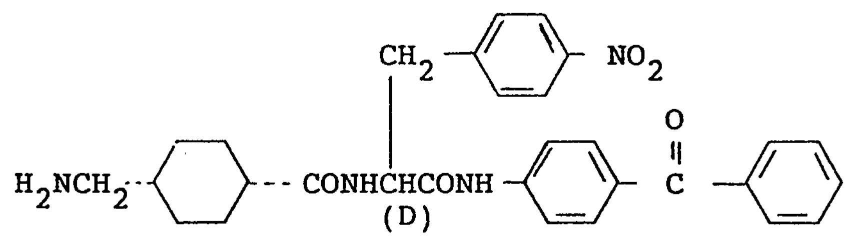 | HCl |

Table 1-2

| Compound No. | Property |
|---|---|
| 1 | NMR:<br>$CD_3OD$,TMS<br>δ 1.93 - 2.00 (9H,m), 2.20 - 2.40 (1H,m), 2.78 (2H,d), 2.90 - 8.20 (2H,m), 4.68 (1H,m), 5.02 (2H,s), 6.84 - 7.40 (9H,m), 8.00 (1H,m), 8.44 - 8.60 (2H,m), 9.32 (1H,s) |
| 2 | NMR:<br>20%$CD_3OD$,TMS<br>δ 0.80 - 2.20 (10H,m), 2.52 (2H,d), 2.60 (3H,s), 2.90 - 3.24 (2H,d), 4.76 (1H,m), 7.12 - 7.96 (9H,m) |
| 3 | NMR:<br>$CD_3OD$,TMS<br>δ 0.88 - 2.36 (10H,m), 2.56 (3H,s), 2.72 (3H,s), 2.76 - 3.22 (4H,m), 4.64 - 4.76 (1H,m), 5.02 (2H,s), 6.84 - 7.96 (12H,m) |
| 4 | NMR:<br>$CD_3OD$,TMS<br>δ 0.94 - 2.32 (10H,m), 2.76 - 3.25 (4H,m), 4.64 - 4.72 (1H,m), 5.01 (2H,s), 6.84 - 8.60 (13H,m) |
| 5 | NMR:<br>$CD_3OD$,TMS<br>δ 0.94 - 2.40 (10H,m), 2.60 (3H,s), 2.80 - 3.30 (4H,m), 4.70 (1H,m), 5.44 (2H,s), 6.98 - 8.88 (12H,m) |

Table 1-2 (continued)

| Compound No. | Property |
|---|---|
| 6 | NMR:<br>$CD_3OD$,TMS<br>δ 0.96 - 2.40 (10H,m), 2.56 (3H,s), 2.80 -3.20 (4H,m),<br>4.66 - 4.77 (1H,m), 5.55 (2H,s), 7.04 - 8.90 (12H,m) |
| 7 | NMR:<br>$CD_3OD$,TMS<br>δ 0.80 -2.32 (10H,m), 2.50 (3H,s), 2.78 (2H,d), 2.90 - 3.30 (2H,m),<br>4.70 - 4.82 (1H,m), 6.76 -7.96 (13H,m) |
| 8 | NMR:<br>$CD_3OD$,TMS<br>δ 0.86 - 2.35 (10H,m), 2.70 - 3.15 (4H,m), 3.55 (2H,s),<br>4.62 -4.72 (1H,m), 5.01 (2H,s), 6.84 - 7.45 (13H,m) |
| 9 | NMR:<br>$CD_3OD$,TMS<br>δ 0.90 - 2.40 (10H,m), 2.58 (3H,s), 2.80 (2H,d) 3.10 (2H,m),<br>7.0 -8.90 (1H,m) |
| 10 | NMR:<br>$CD_3OD$,TMS<br>δ 0.90 - 2.20 (10H,m), 2.75 (2H,d), 3.60 - 3.70 (2H,m), 4.85 (1H,t),<br>7.30 - 7.90 (11H,m), 8.15 (2H,d) |

Table 1-2 (continued)

| Compound No. | Property |
|---|---|
| 11 | NMR:<br>$CD_3OD$,TMS<br>δ 0.88 - 2.40 (10H,m), 2.82 (2H,d), 2.92 - 3.30 (2H,m), 4.70 - 4.82 (1H,m), 6.78 - 7.36 (9H,m), 7.68 - 7.75 (1H,m), 8.03 (1H,d), 8.35 - 8.39 (1H,m) |
| 12 | NMR:<br>$CD_3OD$,TMS<br>δ 0.90 -2.36 (10H,m), 2.80 (2H,d), 3.10 (2H,m), 6.90 - 8.64 (11H,m) |
| 13 | NMR:<br>$CD_3OD$,TMS<br>δ 0.92 - 2.35 (10H,m), 2.80 (2H,d), 2.95 - 3.33 (2H,m), 4.70 - 4.84 (1H,m), 6.86 - 7.36 (9H,m), 8.14 (2H,d), 8.60 (2H,d) |
| 14 | IR:<br>3400, 2940, 2860, 1650, 1555, 1495, 1460, 1250, 1212 |
| 15 | NMR:<br>$CD_3OD$,TMS<br>δ 0.92 - 2.36 (10H,m), 2.76 - 3.22 (4H,m), 4.72 - 4.86 (1H,m), 5.03 (2H,s), 6.77 - 9.16 (12H,m) |
| 16 | IR:<br>3524, 2930, 1715, 1642, 1610, 1537, 1510, 1240 |

Table 1-2 (continued)

| Compound No. | Property |
|---|---|
| 17 | NMR: $CD_3OD$,TMS δ 0.88 - 2.32 (10H,m), 2.75 (2H,d), 2.92 - 3.22 (2H,m), 3.56 (2H,s), 4.70 -4.78 (1H,m), 7.08 - 7.48 (9H,m) |
| 18 | NMR: $CD_3OD$,TMS δ 0.82 - 2.28 (10H,m), 2.65 - 3.20 (4H,m), 3.52 (1H,s), 3.65 (1H,s), 5.00 (2H,m), 6.80 - 7.48 (13H,m) |
| 19 | NMR: $CD_3OD$,TMS δ 2.52 (3H,s), 3.02 - 3.30 (2H,m), 4.16 (2H,s), 5.45 (2H,s), 6.92 - 7.85 (16H,m) |
| 20 | NMR: $CD_3OD$,TMS δ 0.92 - 2.38 (10H,m), 2.80 (2H,d), 2.90 - 3.22 (2H,m), 4.64 - 4.78 (1H,m), 5.49 (2H,s), 6.97 - 8.88 (12H,m) |
| 21 | NMR: $CD_3OD$,TMS δ 0.90 - 2.32 (13H,m), 2.50 - 3.16 (8H,m), 4.07 (2H,q), 4.59 - 4.70 (1H,m), 5.03 (2H,m), 6.82 - 7.42 (13H,m) |

Table 1-2 (continued)

| Compound No. | Property |
| --- | --- |
| 22 | NMR:<br>$CD_3OD$,TMS<br>δ 0.90 - 2.36 (10H,m), 2.77 (3H,s), 2.90 - 3.24 (2H,m), 4.69 - 7.49 (1H,m), 7.08 - 7.32 (5H,m) 7.77 (4H,dd) |
| 23 | NMR:<br>$CD_3OD$,TMS<br>δ 0.95 - 2.32 (10H,m), 2.76 - 3.25 (4H,m), 4.64 - 4.73 (1H,m), 5.02 (2H,s), 6.84 - 8.61 (13H,m) |
| 24 | NMR:<br>$CD_3OD$,TMS<br>δ 0.88 - 2.36 (10H,m), 2.55 (3H,s), 2.78 (2H,d), 2.88 - 3.19 (2H,m), 4.61 - 4.75 (1H,m), 5.49 (2H,s), 6.92 - 8.84 (12H,m) |

Table 2

| Compound | Plasmin | | Trypsin | Plasma kallikrein | Urokinase | FXa |
|---|---|---|---|---|---|---|
| No. | S-2251 | Fibrin | S-2238 | S-2302 | S-2444 | S-2222 |
| 1 | 6.1 | 2.9 | 4.4 | 1.7 | 45 | |
| 2 | 36 | 21 | 1.3 | 0.85 | 58 | 540 |
| 3 | 0.8 | 0.1 | 1.0 | 0.38 | 40 | 35 |
| 4 | 3.4 | 0.7 | | 1.2 | 32 | 90 |
| 5 | 1.2 | 0.7 | 1.0 | 1.2 | 25 | 65 |
| 6 | 2.7 | 1.4 | 2.4 | 2.0 | 75 | 160 |
| 7 | 19 | 14 | 2.3 | 0.38 | 110 | >200 |
| 8 | 25 | 9.2 | 5.3 | 3.0 | 260 | 290 |
| 9 | 1.0 | 0.4 | 0.2 | 0.18 | 19 | 27 |
| 10 | 7.0 | 1.8 | 0.08 | 2.7 | 5.0 | |
| 11 | 31 | 10 | 2.3 | 0.43 | 190 | |
| 12 | 0.9 | 0.6 | 1.2 | 0.66 | 37 | 68 |
| 13 | 65 | 24 | 8.8 | 0.83 | 87 | >200 |

Table 2 (continued)

| Compound | Plasmin | | Trypsin | Plasma kallikrein | Urokinase | FXa |
|---|---|---|---|---|---|---|
| No. | S-2251 | Fibrin | S-2238 | S-2302 | S-2444 | S-2222 |
| 14 | 98 | 70 | 15 | 2.0 | 250 | |
| 15 | 15 | 6.8 | 5.7 | 5.1 | 110 | |
| 16 | 12 | 9.0 | 14 | 1.8 | 210 | |
| 17 | 760 | 460 | 12 | 1.8 | 610 | >1000 |
| 18 | 36 | 31 | 14 | 3.6 | >500 | |
| 19 | 4.2 | 2.5 | 4.2 | 12 | | |
| 20 | 65 | 32 | 24 | 1.4 | | 700 |
| 21 | | | 0.8 | | | |
| 22 | >1000 | 560 | 26 | 2.7 | >1000 | >400 |
| 23 | 120 | 140 | 160 | 360 | >1000 | |
| 24 | 78 | 240 | 230 | >1000 | >1000 | |
| 25 | 240 | 58 | 8.5 | >250 | 170 | |

Table 3-1 Existing control drug

| No. | Compound | |
|---|---|---|
| 1 | Aprotinin | |
| 2 | FOY 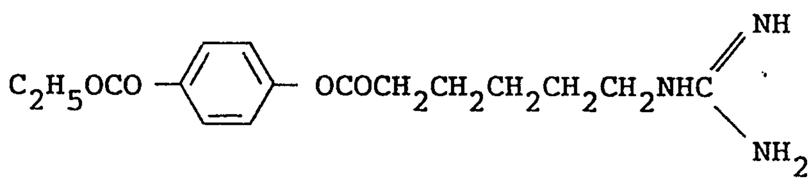 | $CH_3SO_3H$ |
| 3 | FUT-175 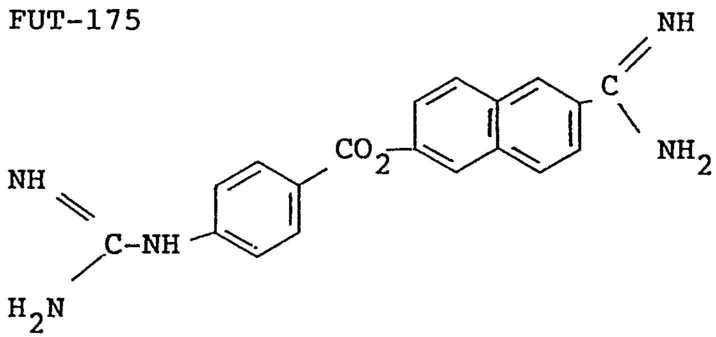 | $2CH_3SO_3H$ |

Table 3-2

| No. (Compound) | Plasmin S-2251 | Plasmin Fibrin | Trypsin S-2238 | Plasma kallikrein S-2302 | Urokinase S-2444 | FXa S-2238 |
|---|---|---|---|---|---|---|
| 1 (Aprotinin) μ/ml | 2.5 | 1.6 | 3.5 | 30 | >1000 | >1000 |
| 2 (FOY) μmol | 3.4 | 3.0 | 7.0 | 1.3 | 1.3 | 43 |
| 3 (FUT-175) μmol | 0.43 | 0.22 | 0.27 | 0.08 | 0.01 | 5.8 |

Table 4-1

| Compound No. | Effect on experimental pancreatitis Survival ratio(%) after 24 hours and 48 hours at dose of 80 μg/kg/min. x 80 min. | |
|---|---|---|
| | After 24 hours | After 48 hours |
| control | 22.7 | 13.6 |
| 1 | 55.5 | 29.1 |
| 5 | 43.3 | 26.7 |
| 6 | 62.5 | 54.1 |
| 8 | 58.3 | 58.3 |
| 9 | 66.7 | 58.3 |
| 10 | 50.0 | 33.3 |
| 11 | 42.0 | 26.7 |
| 12 | 58.3 | 41.7 |
| 14 | 58.3 | 41.7 |
| 15 | 50.0 | 25.0 |
| 16 | 58.3 | 58.3 |
| 17 | 46.4 | 37.3 |
| 18 | 41.7 | 33.3 |
| 19 | 50.0 | 33.3 |
| 20 | 58.3 | 41.7 |
| 21 | 45.5 | 36.4 |

Table 4-2

| Compound No. | Effect on experimental pancreatitis (100 mg/kg p.o.) | |
|---|---|---|
| | After 24 hours | After 48 hours |
| control | 28.3 | 21.6 |
| 6 | 66.7 | 53.3 |
| 8 | 58.3 | 33.3 |
| 9 | 76.9 | 53.8 |
| 10 | 66.7 | 53.3 |
| 20 | 58.3 | 41.7 |

Table 4-3

| Effect of existing control drug on experimental pancreatitis | | | | |
|---|---|---|---|---|
| Compound No. | Name of Compound | Does | After 24 hours | After 24 hours |
| Control | - | - | 22.7 | 13.6 |
| 2 | FOY | 250 μg/kg/min x 80 min | 16.6 | 8.3 |
| | | 1000 μg/kg/min x 80 min | 42.0 | 16.5 |
| 3 | FUT-175 | 50 μg/kg/min x 80 min | 33.3 | 25.0 |

Table 5-1

| Effect on experimental DIC and MOF | | | | | | | |
|---|---|---|---|---|---|---|---|
| Compound No. | Dose (μg/kg/min) | Plat x $10^4$/μl | Fbg mg/dl | FDP μg/ml | GOT U/ml | CPK U/1 | BUN mg/dl |
| Normal | - | 117 ± 12 | 281 ± 21 | 1 - 21 | 125 ± 6 | 8 ± 1 | 12 ± 2 |
| Control | - | 45 ± 5 | 71 ± 12 | 32 - 256 | 378 ± 51 | 35 ± 5 | 37 ± 2 |
| 1 | 10 | 65 ± 6 | 146 ± 7 | 16 - 32 | 299 ± 63 | | 28 ± 3 |
| 3 | 10 | 78 ± 7 | 160 ± 13 | 8 - 64 | 133 ± 4 | 14 ± 2 | 40 ± 5 |
| 6 | 1 | 68 ± 11 | 140 ± 18 | 32 - 65 | 217 ± 29 | | 36 ± 2 |
| | 10 | 95 ± 14 | 179 ± 15 | 8 - 32 | 196 ± 28 | 22 ± 6 | 26 ± 2 |
| 9 | 10 | 59 ± 8 | 117 ± 22 | | 304 ± 37 | | 34 ± 4 |
| | 100 | 53 ± 10 | 147 ± 22 | | 222 ± 56 | 25 ± 3 | 32 ± 4 |
| 10 | 10 | 68 ± 8 | 109 ± 30 | 16 - 128 | 302 ± 29 | | 32 ± 2 |
| | 100 | 66 ± 10 | 128 ± 32 | 16 - 128 | 258 ± 60 | 20 ± 5 | 30 ± 2 |
| 17 | 100 | 63 ± 2 | 143 ± 23 | 16 - 64 | 146 ± 21 | 22 ± 6 | 34 ± 11 |
| 19 | 100 | 80 ± 11 | 147 ± 16 | 16 - 32 | 219 ± 39 | 19 ± 2 | 30 ± 4 |
| 22 | 100 | 62 ± 12 | 107 ± 18 | 32 - 64 | 195 ± 35 | 14 ± 3 | 30 ± 3 |

Table 5-2

Effect of existing control drug on experimental DIC and MOF

| Compound No. | Dose ($\mu g/kg/min$) | Plat $\times 10^4/\mu l$ | Fbg mg/dl | FDP $\mu g/ml$ | GOT U/ml | CPK U/l | BUN mg/dl |
|---|---|---|---|---|---|---|---|
| Normal | - | 117 ± 12 | 281 ± 21 | 1 - 21 | 125 ± 6 | 8 ± 1 | 12 ± 2 |
| Control | - | 45 ± 5 | 71 ± 12 | 32 - 256 | 378 ± 51 | 35 ± 5 | 37 ± 2 |
| FOY | 10 | 58 ± 10 | 90 ± 27 | 16 - 128 | 332 ± 67 | 38 ± 7 | 42 ± 5 |
| | 100 | 71 ± 10 | 167 ± 36 | 32 - 64 | 296 ± 54 | 40 ± 2 | 43 ± 5 |
| | 1000 | 34 ± 8 | 37 ± 20 | 32 - 256 | 426 ± 94 | 35 ± 2 | 37 ± 2 |
| FUT-175 | 1 | 35 ± 5 | 77 ± 8 | 64 - 128 | 300 ± 31 | 37 ± 17 | 34 ± 2 |
| | 10 | 66 ± 11 | 27 ± 6 | 32 - 128 | 346 ± 90 | 48 ± 14 | 32 ± 1 |
| | 100 | 24 ± 4 | 69 ± 18 | 16 - 64 | 425 ± 69 | 50 ± 10 | 34 ± 5 |

Table 6-1

| Compound No. | Effect on experimental shock (Survival ratio (%) after 24 hours at dose of 30 mg/kg) |
|---|---|
| Control | 35 |
| 1 | 82 |
| 2 | 50 |
| 3 | 67 |
| 4 | 82 |
| 6 | 70 |
| 7 | 90 |
| 10 | 79 |
| 11 | 84 |
| 12 | 67 |
| 13 | 79 |
| 23 | 51 |
| 25 | 70 |

Table 6-2

| Effect of existing control drug on experimental shock | | |
|---|---|---|
| Compound | Dose | Survival ratio (%) after 24 hours |
| Control | - | 35 |
| trasylol | 100,000 U/kg | 53 |
| FUT-175 | 30 mg/kg | 26 |

Table 7-1

| Compound No. | Acute toxicity ($LD_{50}$ mg/kg) |
|---|---|
| 1 | 70 |
| 2 | 50 |
| 3 | >100 |
| 4 | 85 |
| 5 | 50 |
| 6 | 85 |
| 7 | 60 |
| 8 | >100 |
| 9 | 60 |
| 10 | 85 |
| 11 | >100 |
| 12 | 60 |
| 13 | 85 |
| 14 | 50 |
| 15 | >100 |
| 16 | >100 |
| 17 | >100 |
| 18 | >100 |
| 20 | >100 |
| 21 | 100 |
| 22 | >100 |
| 23 | 60 |

Table 7-2

| Acute toxicity of existing control drug | |
|---|---|
| Name of drug | ($LD_{50}$ mg/kg) |
| FOY | 248 |
| FUT-175 | 24 |

Table 8-1

| Compound No. | Half life in blood (min.) |
|---|---|
| 1 | 15.3 |
| 6 | 12.2 |
| 8 | 10.6 |
| 10 | 6.8 |
| 12 | 8.0 |
| 20 | 14.0 |

Table 8-2

| Name of drug | Half life of existing control drug (min.) |
|---|---|
| FOY | 0.4 |
| FUT-175 | 2 - 3 |

CLAIMS

1. A pharmaceutical preparation for pancreatitis and the like containing, as an effective ingredient, a compound represented by the general formula:

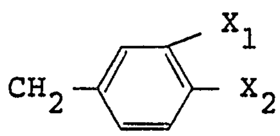

A-CONHCHCONH-Y

wherein A represents $H_2NCH_2$ 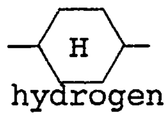 or $H_2NCH_2$ 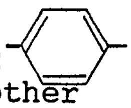 ; one of $X_1$ and $X_2$ represents hydrogen and the other represents hydrogen, nitro, phenoxy (optionally substituted by nitro or by halogen), benzyloxy, picolyloxy or pyridyloxy (optionally substituted by nitro); and Y represents $C_1$-$C_6$ alkyl, phenyl (optionally substituted by $C_1$-$C_4$ alkylcarbonyl, by phenylcarbonyl, by carboxyl, or by $C_1$-$C_4$ alkyl optionally substituted by carboxyl or $C_1$-$C_4$ alkoxycarbonyl), pyridyl (optionally substitued by halogen), or pyrimidyl, or pharmaceutically acceptable salts thereof.

# INTERNATIONAL SEARCH REPORT

International Application No PCT/JP89/00567

**I. CLASSIFICATION OF SUBJECT MATTER** (if several classification symbols apply, indicate all) [6]

According to International Patent Classification (IPC) or to both National Classification and IPC

Int. $Cl^4$ A61K31/165, A61K31/195, A61K31/235, A61K31/44, A61K31/505

**II. FIELDS SEARCHED**

Minimum Documentation Searched [7]

| Classification System | Classification Symbols |
|---|---|
| IPC | A61K31/165, A61K31/195, A61K31/235, A61K31/44, A61K31/505 |

Documentation Searched other than Minimum Documentation to the Extent that such Documents are Included in the Fields Searched [8]

**III. DOCUMENTS CONSIDERED TO BE RELEVANT** [9]

| Category * | Citation of Document, [11] with indication, where appropriate, of the relevant passages [12] | Relevant to Claim No. [13] |
|---|---|---|
| X | JP, A, 63-22061 (Showa Denko Kabushiki Kaisha) 29 January 1988 (29. 01. 88) Claim, page 3, lower left column, lines 2 to 3, page 48, lower left column, line 15 to lower right column, line 4, pages 49 to 50, Table 2 & EP, A, 217,286 | 1 |
| P | JP, A, 63-239256 (Showa Denko Kabushiki Kaisha) 5 October 1988 (05. 10. 88) Claim, page 3, upper right column, lines 8 to 18, page 42, lower left column, lines 6 to 11, pages 44 to 45, Table 2 (Family: none) | 1 |
| P | EP, A, 284,632 (Showa Denko K.K.) 5 October 1988 (05. 10. 88) | 1 |
| A | EP, A, 183,271 (Showa Denko K.K.) 4 June 1986 (04. 06. 86) & JP, A, 61-189255 & JP, A, 61-218565 & JP, A, 62-5945 | 1 |

* Special categories of cited documents: [10]

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance: the claimed invention cannot be considered novel or cannot be considered to involve an inventive step

"Y" document of particular relevance: the claimed invention cannot be considered to involve an inventive step when the document is combined with one or more other such documents, such combination being obvious to a person skilled in the art

"&" document member of the same patent family

**IV. CERTIFICATION**

| Date of the Actual Completion of the International Search | Date of Mailing of this International Search Report |
|---|---|
| August 22, 1989 (22. 08. 89) | September 4, 1989 (04. 09. 89) |
| **International Searching Authority** | **Signature of Authorized Officer** |
| Japanese Patent Office | |

FURTHER INFORMATION CONTINUED FROM THE SECOND SHEET

| | | |
|---|---|---|
| A | JP, A, 62-72656 (Showa Denko Kabushiki Kaisha)<br>3 April 1987 (03. 04. 87)<br>Claim, page 2, upper right column, lines 18 to 19<br>(Family: none) | 1 |
| A | JP, A, 62-6681 (Showa Denko Kabushiki Kaisha)<br>13 January 1987 (13. 01. 87)<br>Claim, page 2, upper right column, line 6<br>(Family: none) | 1 |

V.☐ **OBSERVATIONS WHERE CERTAIN CLAIMS WERE FOUND UNSEARCHABLE** [1]

This international search report has not been established in respect of certain claims under Article 17(2) (a) for the following reasons:

1.☐ Claim numbers ..........., because they relate to subject matter not required to be searched by this Authority, namely:

2.☐ Claim numbers ..........., because they relate to parts of the international application that do not comply with the prescribed requirements to such an extent that no meaningful international search can be carried out, specifically:

3.☐ Claim numbers ..........., because they are dependent claims and are not drafted in accordance with the second and third sentences of PCT Rule 6.4(a).

VI.☐ **OBSERVATIONS WHERE UNITY OF INVENTION IS LACKING** [2]

This International Searching Authority found multiple inventions in this international application as follows:

1.☐ As all required additional search fees were timely paid by the applicant, this international search report covers all searchable claims of the international application.

2.☐ As only some of the required additional search fees were timely paid by the applicant, this international search report covers only those claims of the international application for which fees were paid, specifically claims:

3.☐ No required additional search fees were timely paid by the applicant. Consequently, this international search report is restricted to the invention first mentioned in the claims; it is covered by claim numbers:

4.☐ As all searchable claims could be searched without effort justifying an additional fee, the International Searching Authority did not invite payment of any additional fee.

Remark on Protest

☐ The additional search fees were accompanied by applicant's protest.

☐ No protest accompanied the payment of additional search fees.

International Application No. PCT/JP89/00567

FURTHER INFORMATION CONTINUED FROM THE SECOND SHEET

| | | |
|---|---|---|
| A | JP, A, 61-1651 (Toyama Chemical Co., Ltd.) 7 January 1986 (07. 01. 86) & US, A, 4,610,983 | 1 |

## V. ☐ OBSERVATIONS WHERE CERTAIN CLAIMS WERE FOUND UNSEARCHABLE [1]

This international search report has not been established in respect of certain claims under Article 17(2) (a) for the following reasons:

1. ☐ Claim numbers ...... ....., because they relate to subject matter not required to be searched by this Authority, namely:

2. ☐ Claim numbers ..........., because they relate to parts of the international application that do not comply with the prescribed requirements to such an extent that no meaningful international search can be carried out, specifically:

3. ☐ Claim numbers ..........., because they are dependent claims and are not drafted in accordance with the second and third sentences of PCT Rule 6.4(a).

## VI. ☐ OBSERVATIONS WHERE UNITY OF INVENTION IS LACKING [2]

This International Searching Authority found multiple inventions in this international application as follows:

1. ☐ As all required additional search fees were timely paid by the applicant, this international search report covers all searchable claims of the international application.
2. ☐ As only some of the required additional search fees were timely paid by the applicant, this international search report covers only those claims of the international application for which fees were paid, specifically claims:
3. ☐ No required additional search fees were timely paid by the applicant. Consequently, this international search report is restricted to the invention first mentioned in the claims; it is covered by claim numbers:
4. ☐ As all searchable claims could be searched without effort justifying an additional fee, the International Searching Authority did not invite payment of any additional fee.

Remark on Protest

☐ The additional search fees were accompanied by applicant's protest.

☐ No protest accompanied the payment of additional search fees.